# EUROPEAN PATENT APPLICATION

(11) **EP 3 461 488 A1**
(43) Date of publication of application: **03.04.2019**
(21) Application number: 17193557.0
(22) Date of filing: 27.09.2017
(51) Int. Cl.: A61K 31/713, A61P 35/00

(54) **COMBINATION OF A DBAIT MOLECULE AND A HDAC INHIBITOR FOR TREATING CANCER**

(71) Applicant: ONXEO, 75015 Paris (FR)
(72) Inventor: The designation of the inventor has not yet been filed
(74) Representative: Cabinet Becker et Associés

(57) **Abstract**

The present invention relates to the combination of a Dbait acid molecule with a HDAC inhibitor for treating cancer.

SEQUENCE LISTING

<110>  ONXEO

<120>  COMBINATION OF A DBAIT MOLECULE AND A HDAC INHIBITOR FOR TREATING
        CANCER

<130>  PAT290

<160>  6

<170>  PatentIn version 3.5

<210>  1
<211>  32
<212>  DNA
<213>  artificial sequence

<220>
<223>  Dbait32

<400>  1
acgcacgggt gttgggtcgt ttgttcggat ct                                    32


<210>  2
<211>  32
<212>  DNA
<213>  artificial sequence

<220>
<223>  Dbait32Ha

<400>  2
cgtaggtctg tttggtggct ttgcagtggc ac                                    32


<210>  3
<211>  32
<212>  DNA
<213>  artificial sequence

<220>
<223>  Dbait32Hb

<400>  3
gctaggcttg tttgctgggt tgtaggcaca gc                                    32


<210>  4
<211>  32
<212>  DNA
<213>  artificial sequence

<220>
<223>  Dbait32Hc

<400>  4
gctgtgccca caacccagca aacaagccta ga                                    32


<210>  5
<211>  32

<212>  DNA
<213>  artificial sequence

<220>
<223>  Dbait32Hd

<400>  5
gctaggtctg tttggtggct ttgcagtggc ac                                    32


<210>  6
<211>  32
<212>  DNA
<213>  artificial sequence

<220>
<223>  Dbait molecule IIa



<220>
<221>  misc_feature
<223>  at the 3′ end of the complementary strand the three last
       nucleotides with phosphorothioate or methylphosphonate backbone

<220>
<221>  misc_feature
<222>  (1)..(1)
<223>  at the 5′ end, Lm = carboxamido oligoethylene glycol + C = single
       or double chain fatty acids, cholesterol, sugars, peptides or
       proteins

<220>
<221>  misc_feature
<222>  (1)..(1)
<223>  at the 5′ end, Lm = tetraethyleneglycol + C = cholesterol

<220>
<221>  stem_loop
<222>  (1)..(32)

<220>
<221>  modified_base
<222>  (1)..(3)
<223>  mod_base= phosphorothioate or methylphosphonate backbone

<220>
<221>  misc_feature
<222>  (32)..(32)
<223>  loop L′ =
       1,19-bis(phospho)-8-hydraza-2-hydroxy-4-oxa-9-oxo-nonadecane

<400>  6
gctgtgccca caacccagca aacaagccta ga                                    32

## Description

### Field of the Invention

The present invention relates to the field of medicine, in particular of oncology.

### Background of the Invention

Histone deacetylases (HDACs) are now known to target both histone proteins and many non-histone proteins by removing acetyl groups from these proteins. There are currently 18 known HDACs, which are classified into four groups. Class I HDACs, includes HDAC1-3 and HDAC8. Class II HDACs include HDAC4-7 10and HDAC9-10. Class III HDACs (also known as the sirtuins) include SIRT1-7. Class IV HDACs, which contains only HDAC11, has features of both Class I and II HDACs. These posttranslational modifications contribute to the regulation of numerous essential biological processes in eukaryotes including transcriptional regulation, cell cycle progression and apoptosis. More importantly HDACs are frequently dysregulated in cancer.

Therefore, HDAC inhibitors (HDACIs) have been shown to exert multiple cytotoxic actions in cancer cells. This observation led to the development of HDACIs to treat cancers. However, despite their preclinical potential, the clinical use of HDACIs remains restricted to certain subsets of T-cell lymphoma and multiple myeloma. Vorinostat (SAHA; Zolinza® MSD, USA), romidepsin (FK228; Istodax®; Celgene, USA), and belinostat (PXD-101; Beleodaq® Spectrum Pharmaceuticals, Inc., USA) are approved for cutaneous or peripheral T-cell lymphoma, while panobinostat (LBH-589; Faridak® Novartis, Switzerland) is approved for combination therapy of multiple myeloma. HDACIs are effective against hematological malignancies, however ineffective against solid tumors. The use of HDAC inhibitors is thus limited to particular tumor types and cannot be used for treating any cancer.

As HDAC is involved in many cellular processes, the mechanism of action of HDACIs in tumor cells remains not completely elucidated. Moreover, since most of the HDACIs studied so far have little or no HDAC isoform specificity and have several non-histone protein targets their administration can initiate alterations in several biochemical or physiological functions or pathways leading to some of toxicity or side effects. The development of more specific HDACIs have been proposed to overcome this problem and also enhance therapeutic gains with a better understanding of the underlying mechanism(s).

Indeed, there remains a substantial unmet need for therapeutics that have better therapeutic efficacy, longer clinical benefit, and improved safety profiles, particularly for those patients with cancers that are resistant to existing therapeutics such as triple-negative breast cancer.

### Summary of the Invention

The present invention provides a combined treatment allowing to use HDAC inhibitors for treating any kind of cancers, in particular for solid cancer, especially resistant solid cancer such as triple-negative breast cancer (TNBC) without displaying toxicity towards normal cells.

In addition, the present invention provides a combination of HDAC inhibitors with Dbait acid molecules, leading to a synergistic effect against tumors.

The present invention relates to a pharmaceutical composition or a kit (kit-of-parts) comprising a HDAC inhibitor and a Dbait molecule, in particular for use for treating cancer.

The present invention also relates to a HDAC inhibitor for use for treating cancer in combination with a Dbait molecule or to a Dbait molecule as defined herein for use for treating cancer in combination with a HDAC inhibitor.

It further relates to a kit (kit-of-parts) comprising a HDAC inhibitor and a Dbait molecule as a combined preparation for simultaneous, separate or sequential use, in particular in the treatment of cancer.

It further relates to a method for treating a cancer in a subject in need thereof, comprising administering an amount (therapeutically or subtherapeutically effective amount) of Dbait molecule and an amount (therapeutically or subtherapeutically effective amount) of a HDAC inhibitor.

Preferably, the Dbait molecule has at least one free end and a DNA double stranded portion of 20-200 bp with less than 60% sequence identity to any gene in a human genome.

More preferably, the Dbait molecule has one of the following formulae: 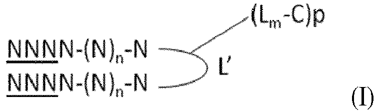 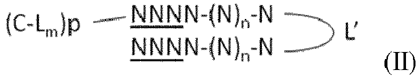 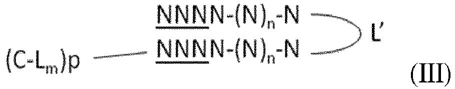 wherein N is a deoxynucleotide, n is an integer from 1 to 195, the underlined N refers to a nucleotide having or not a modified phosphodiester backbone, L' is a linker, C is a molecule facilitating endocytosis preferably selected from a lipophilic molecule and a ligand which targets cell receptor enabling receptor mediated endocytosis, L is a linker, m and p, independently, are an integer being 0 or 1.

In a very specific embodiment, the Dbait molecule has the following formula (AsiDNA):

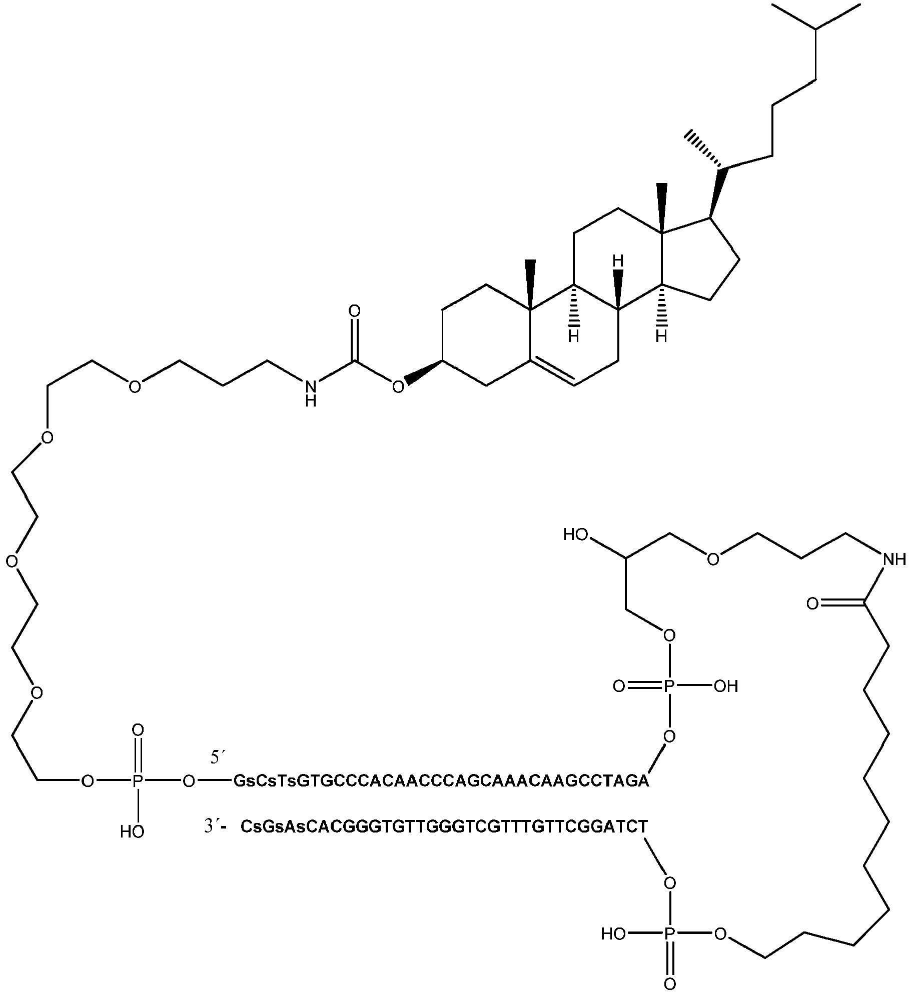

Preferably, the HDAC inhibitor is selected from the group consisting of belinostat (PXD-101), vorinostat (SAHA), entinostat (MS-275) and romidepsin (FK-228).

In a preferred embodiment, the HDACi inhibitor is a hydroxamic acid HDAC inhibitor.

In another preferred embodiment, the HDACi inhibitor is a pan-HDAC inhibitor, preferably a hydroxamic acid pan-HDAC inhibitor.

In a particular aspect, the HDAC inhibitor is used with a sub-therapeutic amount.

All cancer type can be treated. More preferably, the cancer is selected from leukemia, lymphoma, sarcoma, melanoma, and cancers of the head and neck, kidney, ovary, pancreas, prostate, thyroid, lung, in particular small-cell lung cancer, and non-small-cell lung cancer, esophagus, breast (TBNC), bladder, colorectum, liver, cervix, and endometrial and peritoneal cancers. In particular, the cancer is a solid cancer. In a particular aspect, the cancer is a metastatic cancer or high-grade or advanced cancer. In a particular embodiment, the cancer is selected from leukemia, lymphoma, melanoma, sarcoma, cancer of the head and neck, breast cancer (TBNC), brain cancer, colorectum cancer, and cancer of cervix.

### Brief Description of the Drawings

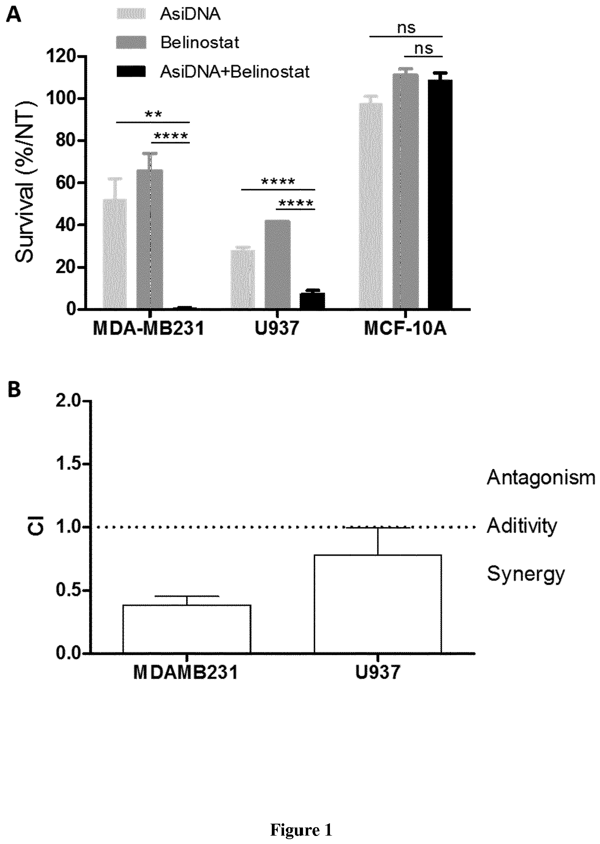

**Figure 1****. The combined treatment AsiDNA and belinostat display synergistic efficacy. A.** Efficacy of AsiDNA, belinostat or both (at doses corresponding to IC50) was monitored 7 days or 4 days after treatment by XTT assay. Survival is expressed as percentage of living cells relative to non-treated condition. Data are expressed as mean + SD of at least 4 independent cultures. **B.** Combination index (CI) analysis to determine synergy between AsiDNA and belinostat was carried out using Calcusyn software as described in methods. CI<1 indicates synergy, CI=1 is additive, and CI>1 indicates antagonism. **, P<0.01; ***, P<0.001; ****, P<0.0001; ns, not significant.

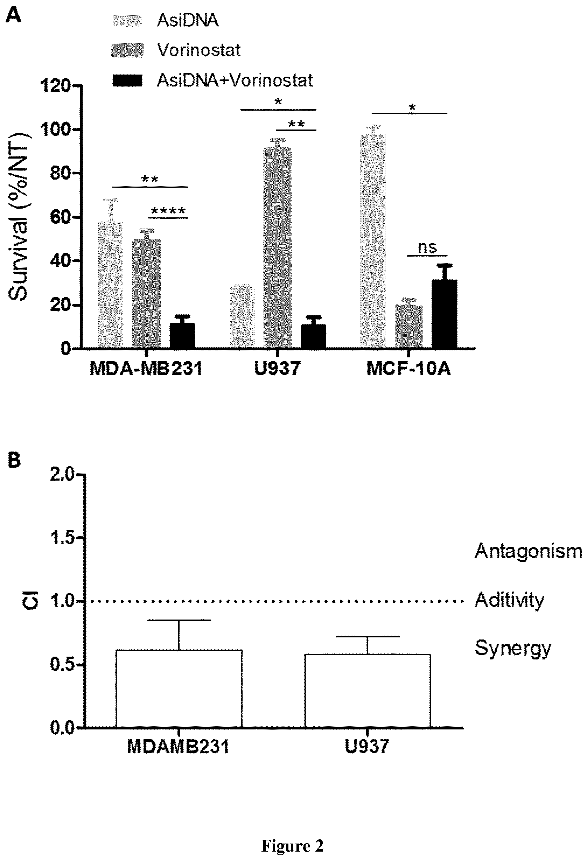

**Figure 2****. The combined treatment AsiDNA and vorinostat display synergistic efficacy. A.** Efficacy of AsiDNA, vorinostat or both (at doses corresponding to IC50) was monitored 7 days or 4 days after treatment by XTT assay. Survival is expressed as percentage of living cells relative to non-treated condition. Data are expressed as mean + SD of at least 4 independent cultures. **B.** Combination index (CI) analysis to determine synergy between AsiDNA and vorinostat was carried out using Calcusyn software as described in methods. CI<1 indicates synergy, CI=1 is additive, and CI>1 indicates antagonism. *, P<0.05; **, P<0.01; ****, P<0.0001; ns, not significant.

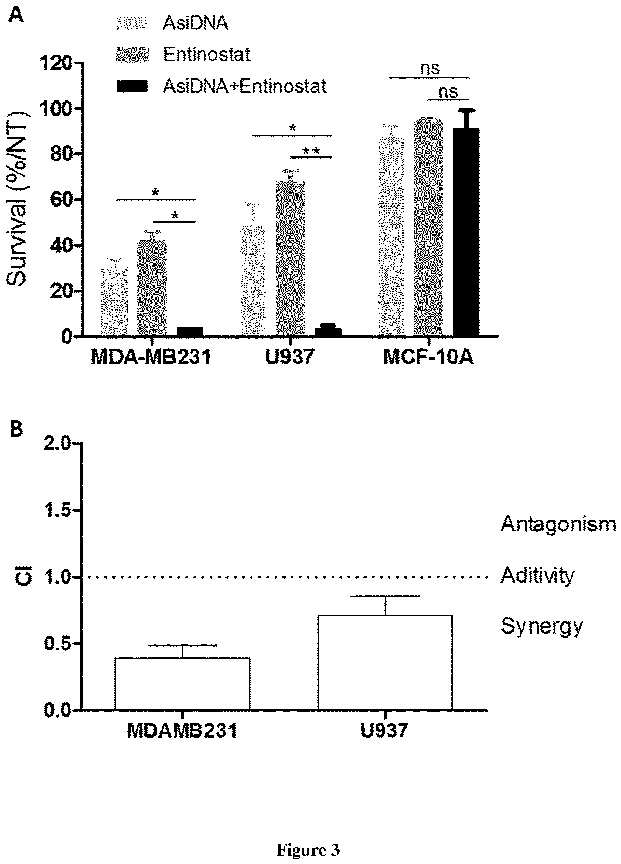

**Figure 3****. The combined treatment AsiDNA and entinostat display synergistic efficacy. A.** Efficacy of AsiDNA, entinostat or both (at doses corresponding to IC50) was monitored 7 days or 4 days after treatment by XTT assay. Survival is expressed as percentage of living cells relative to non-treated condition. Data are expressed as mean + SD of at least 4 independent cultures. **B.** Combination index (CI) analysis to determine synergy between AsiDNA and entinostat was carried out using Calcusyn software as described in methods. CI<1 indicates synergy, CI=1 is additive, and CI>1 indicates antagonism. *, P<0.05; **, P<0.01; ****, P<0.0001; ns, not significant.

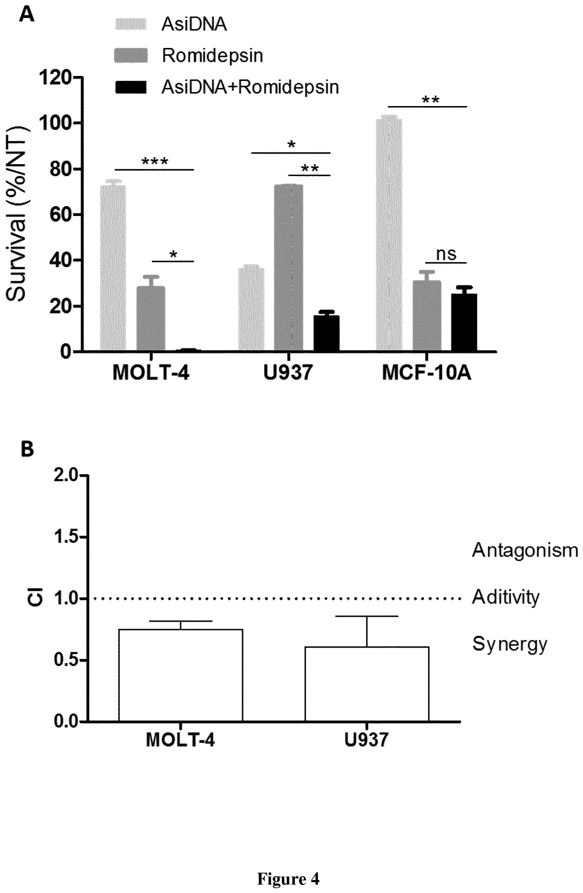

**Figure 4****. The combined treatment AsiDNA and romidepsin display synergistic efficacy. A.** Efficacy of AsiDNA, romidepsin or both (at doses corresponding to IC50) was monitored 7 days or 4 days after treatment by XTT assay. Survival is expressed as percentage of living cells relative to non-treated condition. Data are expressed as mean + SD of at least 4 independent cultures. **B.** Combination index (CI) analysis to determine synergy between AsiDNA and romidepsin was carried out using Calcusyn software as described in methods. CI<1 indicates synergy, CI=1 is additive, and CI>1 indicates antagonism. *, P<0.05; **, P<0.01; ***, P<0.001; ns, not significant.

### Detailed Description of the Invention

The present invention relates to a pharmaceutical composition or a kit (kit-of-parts) comprising a Dbait molecule and a HDAC inhibitor.

The present invention relates to pharmaceutical composition comprising a Dbait molecule and a HDAC inhibitor for use in the treatment of a cancer.

The present invention also relates to kit (kit-of-parts) comprising a Dbait molecule and a HDAC inhibitor and as a combined preparation for simultaneous, separate or sequential use, in particular for use in the treatment of cancer.

The present invention further relates to a method for treating a cancer in a subject in need thereof, comprising administering a therapeutically effective amount of a Dbait molecule and a therapeutically effective amount of a HDAC inhibitor, and optionally a pharmaceutically acceptable carrier.
The present invention further relates to a method for treating a cancer in a subject in need thereof, comprising administering a subtherapeutically effective amount of a Dbait molecule and a subtherapeutically effective amount of a HDAC inhibitor, and optionally a pharmaceutically acceptable carrier.

### Definitions:

The terms "kit", "product" or "combined preparation", as used herein, defines especially a "kit-of-parts" in the sense that the combination partners as defined above can be dosed independently or by use of different fixed combinations with distinguished amounts of the combination partners, i.e. simultaneously or at different time points. The parts of the kit-of-parts can then, e.g., be administered simultaneously or chronologically staggered, that is at different time points and with equal or different time intervals for any part of the kit of parts. The ratio of the total amounts of the combination partners to be administered in the combined preparation can be varied. The combination partners can be administered by the same route or by different routes.

Within the context of the invention, the term treatment denotes curative, symptomatic, preventive treatment as well as maintenance treatment. Pharmaceutical compositions, kits, products and combined preparations of the invention can be used in humans with existing cancer or tumor, including at early or late stages of progression of the cancer. The pharmaceutical compositions, kits, products and combined preparations of the invention will not necessarily cure the patient who has the cancer but will delay or slow the progression or prevent further progression of the disease, ameliorating thereby the patients' condition. In particular, the pharmaceutical compositions, kits, products and combined preparations of the invention reduce the development of tumors, reduce tumor burden, produce tumor regression in a mammalian host and/or prevent metastasis occurrence and cancer relapse. In treating the cancer, the pharmaceutical composition of the invention is administered in a therapeutically effective amount.

By "therapeutically effective amount" it is meant the quantity of the compound of interest of the pharmaceutical composition, kit, product or combined preparation of the invention which prevents, removes or reduces the deleterious effects of cancer in mammals, including humans, alone or in combination with the other active ingredients of the pharmaceutical composition, kit, product or combined preparation. It is understood that the administered dose may be lower for each compound in the composition to the "therapeutically effective amount" define for each compounds used alone or in combination with other treatments than the combination described here. The "therapeutically effective amount" of the composition will be adapted by those skilled in the art according to the patient, the pathology, the mode of administration, etc.

By "subtherapeutically effective amount" it is meant the quantity of the compound of interest of the pharmaceutical composition, kit, product or combined preparation of the invention which is effective at a lower dosage when used in the combination regimen of the invention, as compared to the dosage that is effective when used alone. Subtherapeutically effective amounts may be readily determined by one of skill in the art, in view of the teachings herein. Accordingly, as described herein, subtherapeutically effective amounts of a Dbait molecule (such as AsiDNA) and a HDAC inhibitor (such as belinostat) may be used to achieve a therapeutic effect when administered in combination.

Whenever within this whole specification the terms "treatment of a cancer" or "treating a cancer" or the like are mentioned with reference to the pharmaceutical composition, kit, product or combined preparation of the invention, there is meant: a) a method for treating a cancer, said method comprising administering a pharmaceutical composition, kit, product or combined preparation of the invention to a subject in need of such treatment; b) the use of a pharmaceutical composition, kit, product or combined preparation of the invention for the treatment of a cancer; c) the use of a pharmaceutical composition, kit, product or combined preparation of the invention for the manufacture of a medicament for the treatment of a cancer; and/or d) a pharmaceutical composition, kit, product or combined preparation of the invention for use in the treatment a cancer.

The pharmaceutical compositions, kits, products or combined preparations contemplated herein may include a pharmaceutically acceptable carrier in addition to the active ingredient(s). The term "pharmaceutically acceptable carrier" is meant to encompass any carrier (e.g., support, substance, solvent, etc.) which does not interfere with effectiveness of the biological activity of the active ingredient(s) and that is not toxic to the host to which it is administered. For example, for parental administration, the active compounds(s) may be formulated in a unit dosage form for injection in vehicles such as saline, dextrose solution, serum albumin and Ringer's solution.

The pharmaceutical composition, kit, product or combined preparation can be formulated as solutions in pharmaceutically compatible solvents or as emulsions, suspensions or dispersions in suitable pharmaceutical solvents or vehicle, or as pills, tablets or capsules that contain solid vehicles in a way known in the art. Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion. Formulations suitable for parental administration conveniently comprise a sterile oily or aqueous preparation of the active ingredient which is preferably isotonic with the blood of the recipient. Every such formulation can also contain other pharmaceutically compatible and nontoxic auxiliary agents, such as, e.g. stabilizers, antioxidants, binders, dyes, emulsifiers or flavouring substances. The formulations of the present invention comprise an active ingredient in association with a pharmaceutically acceptable carrier therefore and optionally other therapeutic ingredients. The carrier must be "acceptable" in the sense of being compatible with the other ingredients of the formulations and not deleterious to the recipient thereof. The pharmaceutical compositions, kits, products or combined preparations are advantageously applied by injection or intravenous infusion of suitable sterile solutions or as oral dosage by the digestive tract. Methods for the safe and effective administration of most of these chemotherapeutic agents are known to those skilled in the art. In addition, their administration is described in the standard literature.

### HDAC inhibitors:

The term "histone deacetylase" or "HDAC", as used herein, refers to an enzyme that removes acetyl groups from histones. There are currently 18 known HDACs, which are classified into four groups. Class I HDACs, includes HDAC1-3 and HDAC8. Class II HDACs include HDAC4-7 and HDAC9-10. Class III HDACs (also known as the sirtuins) include SIRT1-7. Class IV HDACs, which contains only HDAC11, has features of both Class I and II HDACs.

The term "histone deacetylase inhibitor" or "HDACi" as used herein, refers to a compound natural or synthetic that inhibits histone deacetylase activity. There exist different classes of HDACi in function of their selectivity for their substrates divided in classical HDACi, selective class I HDACi and selective class II HDACi.

A "classical HDACi" (also known as pan-HDACi) refers thus to a compound natural or not which has the capability to inhibit the histone deacetylase activity independently of the class of HDACs. Therefore a classical HDACi is a non selective HDACi. By "non selective" it is meant that said compound inhibits the activity of classical HDACs (i.e. class I, II and IV) with a similar efficiency independently of the class of HDAC. Examples of classical HDACi include, but are not limited to, Belinostat (PDX-101), Vorinostat (SAHA) and Panobinostat (LBH-589).

A "selective class I HDACi" is selective for class HDACs (i.e. HDAC 1-3 and 8) as compared with class II HDACs (i.e. HDAC4-7, 9 and 10). By "selective" it is meant that selective class I HDACi inhibits class I HDACs at least 5-fold, preferably 10-fold, more preferably 25-fold, still preferably 100-fold higher than class II HDACs. Selectivity of HDACi for class I or class II HDACs may be determined according to previously described method (Kahn et al. 2008). Examples of selective class I HDACi include, but are not limited to, valproic acid (VPA), Romidepsin (FK-228) and Entinostat (MS-275).

A "selective class II HDACi" is selective for class II HDACs (i.e. HDAC4-7, 9 and 10) as compared with class I HDACs (i.e. HDAC 1-3 and 8). By "selective" it is meant that selective class II HDACi inhibits class II HDACs at least 5-fold, preferably 10-fold, more preferably 25-fold, still preferably 100-fold higher than class I HDACs. Examples of selective class II HDACi include, but are not limited to, tubacin and MC-1568 (aryloxopropenyl)pyrrolyl hydroxamate).

HDAC inhibition relies mainly on a mechanism based on the inhibition of the HDAC enzymatic activity which can be determined by a variety of methods well known by the skilled person. Usually, these methods comprise assessing the lysine deacetylase activity of HDAC enzymes using colorimetric HDAC assays. Commercial kits for such techniques are available (see for example, Histone Deacetylase (HDAC) Activity Assay Kit (Fluorometric) purchased from Abcam or Sigma-Aldrich). These methods are ideal for the determination of IC50 values of known or suspected HDAC inhibitors.

Many HDAC inhibitors are known and, thus, can be synthesized by known methods from starting materials that are known, may be available commercially, or may be prepared by methods used to prepare corresponding compounds in the literature.

A preferred class of HDAC inhibitors are hydroxamic acid inhibitors which are disclosed e. g. in WO 97/35990, US-A 5, 369, 108, US-A 5, 608, 108, US-A 5, 700, 811, WO 01/18171, WO 98/55449, WO 93/12075, WO 01/49290, WO 02/26696, WO 02/26703, JP 10182583, WO 99/12884, WO 01/38322, WO 01/70675, WO 02/46144, WO 02/22577 and WO 02/30879. All HDAC inhibitors disclosed in these publications are included herein by reference.

Particularly preferred are HDAC inhibitors are carbamic acid compounds comprising a sulfonamide linkage which are disclosed e. g. in WO 02/30879.

Other HDAC inhibitors which can be included within the compositions of the present invention are cyclic peptide inhibitors, and here it can be referred e. g. to US-A 5,620, 953, US-A 5, 922, 837, WO 01/07042, WO 00/08048, WO 00/21979, WO 99/11659, WO 00/52033 and WO 02/0603. All HDAC inhibitors disclosed in these publications are included herein by reference.

Suitable HDAC inhibitors are also those which are based on a benzamide structure which are disclosed e. g. in Proc. Natl. Acad. Sci. USA (1999), 96: 4592-4597, but also in EP-A 847 992, US 6, 174, 905, JP 11269140, JP 11335375, JP 11269146, EP 974 576, WO 01/38322, WO 01/70675 and WO 01/34131. All HDAC inhibitors, which are disclosed in these documents, are included herein by reference.

The HDAC inhibitors may be used under any pharmaceutically acceptable form, including without limitation, their free form and their pharmaceutically acceptable salts or solvates.

The term "pharmaceutically acceptable salts" refers to salts prepared from pharmaceutically acceptable, preferably non-toxic, bases or acids including mineral or organic acids or organic or inorganic bases. Such salts are also known as acid addition and base addition salts." Examples of pharmaceutically acceptable salts are discussed in Berge et al., 1977, "Pharmaceutically Acceptable Salts," J. Pharm. Sci., Vol. 66, pp. 1-19.

The term "solvate" refers to a molecular complex comprising the drug substance and a stoichiometric or non-stoichiometric amount of one or more pharmaceutically acceptable solvent molecules (e.g., ethanol). The term "hydrate" refers to a solvate comprising the drug substance and a stoichiometric or non-stoichiometric amount of water.

More particularly, examples of suitable HDAC inhibitors according to the invention include, but are not limited to the compounds listed in Table 1 below:

**Table 1: Examples of HDAC inhibitors useful within the context of the invention**

| **Class** | **Agent** |
|---|---|
| Hydroxamates | Belinostat (PDX-101) |
| Hydroxamates | Vorinostat (SAHA) |
| Hydroxamates | Panobinostat (LBH-589) |
| Hydroxamates | Givinostat (IFT2357) |
| Hydroxamates | Abexinostat (PCI-24781) |
| Hydroxamates | Trichostatin A (TSA) |
| Hydroxamates | Quisinostat (JNJ-26481585) |
| Hydroxamates | Pracinostat (SB939) |
| Hydroxamates | Resminostat (4SC-201) |
| Hydroxamates | Tefinostat (CHR-2845) |
| Hydroxamates | Dacinostat (LAQ-824) |
| Hydroxamates | CG200745 |
| Hydroxamates | CHR-3996 |
| Hydroxamates | Citarinostat (ACY-241) |
| Hydroxamates | Ricolinostat (ACY-1215) |
| Hydroxamates | MC-1568 |
| Benzamides | Mocetinostat (MGCD0103) |
| Benzamides | Entinostat (MS-275) |
| Benzamides | Tacedinaline (CI994) |
| Benzamides | Chidamide (CS055/HBI-8000) |
| Benzamides | BML-210 |
| Cyclic peptides | Romidepsin (FK-228, Depsipeptide) |
| Cyclic peptides | Trapoxin A |
| Cyclic peptides | Apicidine |
| Short-chain fatty acids (SCFA) | Sodium Phenylbutyrate |
| Short-chain fatty acids (SCFA) | Valproic acid (VPA) |
| Short-chain fatty acids (SCFA) | Sodium butyrate (S4125) |

In a preferred embodiment, the HDAC inhibitor is selected from the group consisting of belinostat (PXD-101), vorinostat (SAHA), entinostat (MS-275) and romidepsin (FK-228).

Belinostat (also known as PXD-101) has the chemical name (2E)-N-hydroxy-3-[3-(phenylsulfamoyl)phenyl]prop-2-enamide and the following chemical formula:

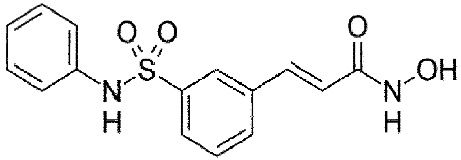

Belinostat is currently commercially available for injection in the U.S. under the brand name Beleodaq® (Spectrum Pharmaceuticals). Typically, liquid formulations of belinostat comprise L-arginine, and are suitable for administration by injection, infusion, intravenous infusion, etc

Belinostat and pharmaceuticals compositions comprising thereof useful in the present combinations are described in the international patent applications N° WO 2002/30879 and WO 2006/120456, the contents of both of which are incorporated herein in their entirety. In certain embodiments, belinostat is formulated with arginine (such as L-arginine).

Vorinostat (also known as suberoylanilide hydroxamic acid (SAHA)) has the chemical name N-hydroxy-N'-phenyloctanediamide the following chemical formula:

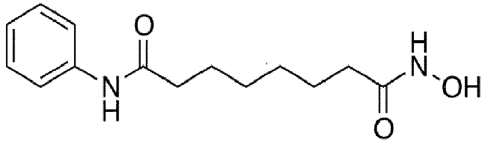

Vorinostat is currently commercially available for oral administration in the U.S. under the brand name Zolinza® (Merck Sharp & Dohme Corp).

Entinostat (also known as MS-275) has the chemical name N-(2- aminophenyl)-4-N-(pyridine-3-yl)methoxycarbonylamino-methyl]-benzamide and has the following chemical formula:

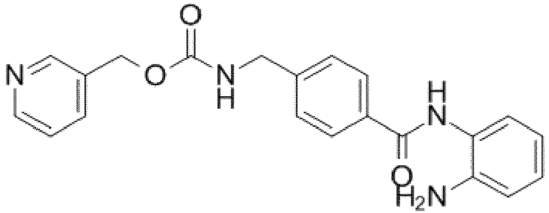

Romidepsin is a natural product which was isolated from Chromobacterium violaceum by Fujisawa Pharmaceuticals. Romidepsin (also known as FK-228) is a bicyclic depsipeptide [1S,4S,7Z,10S,16E,21R)-7-ethylidene-4,21-bis(1methylethyl)-2-oxa-12,13-dithia-5,8,20,23-tetraazabicyclo[8.7.6]tricos-16ene-3,6,9,19,22-pentone] and has the following chemical formula:

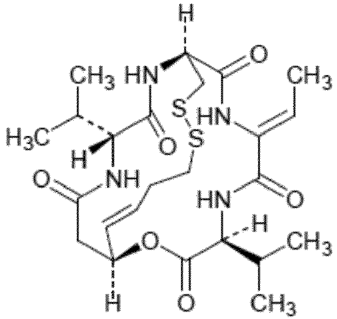

Romidepsin,in is currently commercially available for injection in the U.S. under the brand name Isotodax® (Celgene).

### Dbait molecules:

The term "Dbait molecule" also known as signal interfering DNA (siDNA) as used herein, refers to a nucleic acid molecule, preferably a hairpin nucleic acid molecule, designed to counteract DNA repair. A Dbait molecule has at least one free end and a DNA double stranded portion of 6-200 bp with less than 60% sequence identity to any gene in a human genome.

Preferably, the Dbait molecules for use in the present invention, conjugated or not, can be described by the following formulae: 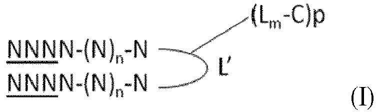 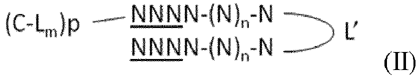 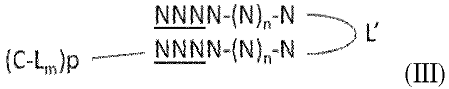 wherein N is a deoxynucleotide, n is an integer from 1 to 195, the underlined N refers to a nucleotide having or not a modified phosphodiester backbone, L' is a linker, C is a molecule facilitating endocytosis preferably selected from a lipophilic molecule and a ligand which targets cell receptor enabling receptor mediated endocytosis, L is a linker, m and p, independently, are an integer being 0 or 1.

In preferred embodiments, the Dbait molecules of formulae (I), (II), or (III) have one or several of the following features:
- N is a deoxynucleotide, preferably selected from the group consisting of A (adenine), C (cytosine), T (thymine) and G (guanine) and selected so as to avoid occurrence of a CpG dinucleotide and to have less than 80% or 70%, even less than 60% or 50% sequence identity to any gene in a human genome.; and/or,
- n is an integer from 1 to 195, preferably from 3 to 195, optionally from 1 to 95, from 2 to 95, from 3 to 95, from 5 to 95, from 15 to 195, from 19-95, from 21 to 95, from 27 to 95, from 1 to 45, from 2 to 35, from 3 to 35, from 5 to 35, from 15 to 45, from 19 to 45, from 21 to 45, or from 27 to 45. In a particularly preferred embodiment, n is 27; and/or,
- the underlined N refers to a nucleotide having or not a phosphorothioate or methylphosphonate backbone, more preferably a phosphorothioate backbone; preferably, the underlined N refers to a nucleotide having a modified phosphodiester backbone; and/or,
- the linker L' is selected from the group consisting of hexaethyleneglycol, tetradeoxythymidylate (T4), 1,19-bis(phospho)-8-hydraza-2-hydroxy-4-oxa-9-oxo-nonadecane; and 2,19-bis(phosphor)-8-hydraza-1-hydroxy-4-oxa-9-oxo-nonadecane and/or,
- m is 1 and L is a carboxamido polyethylene glycol, more preferably carboxamido triethylene or tetraethylene glycol; and/or,
- C is selected from the group consisting of a cholesterol, single or double chain fatty acids such as octadecyl, oleic acid, dioleoyl or stearic acid, or ligand (including peptide, protein, aptamer) which targets cell receptor such as folic acid, tocopherol, sugar such as galactose and mannose and their oligosaccharide, peptide such as RGD and bombesin, and protein such transferring and integrin, preferably is a cholesterol or a tocopherol, still more preferably a cholesterol.

Preferably, C-Lm is a triethyleneglycol linker (10-O-[1-propyl-3-N-carbamoylcholesteryl]-triethyleneglycol radical. Alternatively, C-Lm is a tetraethyleneglycol linker (10-O-[1-propyl-3-N-carbamoylcholesteryl]-tetraethyleneglycol radical.

In a preferred embodiment, the Dbait molecule has the following formula: 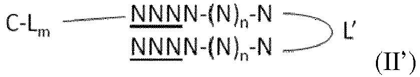 with the same definition than formulae (I), (II), and (III) for N, N, n, L, L', C and m.

In a particular embodiment, the Dbait molecules are those extensively described in PCT patent applications WO2005/040378, WO2008/034866, WO2008/084087 and WO2011/161075, the disclosure of which is incorporated herein by reference.

Dbait molecules may be defined by a number of characteristics necessary for their therapeutic activity, such as their minimal length, the presence of at least one free end, and the presence of a double stranded portion, preferably a DNA double stranded portion. As will be discussed below, it is important to note that the precise nucleotide sequence of Dbait molecules does not impact on their activity. Furthermore, Dbait molecules may contain a modified and/or non-natural backbone.

Preferably, Dbait molecules are of non-human origin (i.e., their nucleotide sequence and/or conformation (e.g., hairpin) does not exist as such in a human cell), most preferably of synthetic origin. As the sequence of the Dbait molecules plays little, if any, role, Dbait molecules have preferably no significant degree of sequence homology or identity to known genes, promoters, enhancers, 5'- or 3'- upstream sequences, exons, introns, and the like. In other words, Dbait molecules have less than 80% or 70%, even less than 60% or 50% sequence identity to any gene in a human genome. Methods of determining sequence identity are well known in the art and include, e.g., Blast. Dbait molecules do not hybridize, under stringent conditions, with human genomic DNA. Typical stringent conditions are such that they allow the discrimination of fully complementary nucleic acids from partially complementary nucleic acids.

In addition, the sequence of the Dbait molecules is preferably devoid of CpG in order to avoid the well-known toll-like receptor-mediated immunological reactions.

The length of Dbait molecules may be variable, as long as it is sufficient to allow appropriate binding of Ku protein complex comprising Ku and DNA-PKcs proteins. It has been showed that the length of Dbait molecules must be greater than 20 bp, preferably about 32 bp, to ensure binding to such a Ku complex and allowing DNA-PKcs activation. Preferably, Dbait molecules comprise between 20-200 bp, more preferably 24-100 bp, still more preferably 26-100, and most preferably between 24-200, 25-200, 26-200, 27-200, 28-200, 30-200, 32-200, 24-100, 25-100, 26-100, 27-100, 28-100, 30-100, 32-200 or 32-100 bp. For instance, Dbait molecules comprise between 24-160, 26-150, 28-140, 28-200, 30-120, 32-200 or 32-100 bp. By "bp" is intended that the molecule comprise a double stranded portion of the indicated length.

In a particular embodiment, the Dbait molecules having a double stranded portion of at least 32 pb, or of about 32 bp, comprise the same nucleotide sequence than Dbait32 (SEQ ID NO: 1), Dbait32Ha (SEQ ID NO: 2), Dbait32Hb (SEQ ID NO: 3), Dbait32Hc (SEQ ID NO: 4) or Dbait32Hd (SEQ ID NO: 5). Optionally, the Dbait molecules have the same nucleotide composition than Dbait32 (SEQ ID NO: 1), Dbait32Ha (SEQ ID NO: 2), Dbait32Hb (SEQ ID NO: 3), Dbait32Hc (SEQ ID NO: 4) or Dbait32Hd (SEQ ID NO: 5) but their nucleotide sequence is different. Then, the Dbait molecules comprise one strand of the double stranded portion with 3 A, 6 C, 12 G and 11 T. Preferably, the sequence of the Dbait molecules does not contain any CpG dinucleotide.

Alternatively, the double stranded portion comprises at least 16, 18, 20, 22, 24, 26, 28, 30 or 32 consecutive nucleotides of Dbait32 (SEQ ID NO: 1), Dbait32Ha (SEQ ID NO: 2), Dbait32Hb (SEQ ID NO: 3), Dbait32Hc (SEQ ID NO: 4) or Dbait32Hd (SEQ ID NO: 5). In a more particular embodiment, the double stranded portion consists in 20, 22, 24, 26, 28, 30 or 32 consecutive nucleotides of Dbait32 (SEQ ID NO: 1), Dbait32Ha (SEQ ID NO: 2), Dbait32Hb (SEQ ID NO: 3), Dbait32Hc (SEQ ID NO: 4) or Dbait32Hd (SEQ ID NO: 5).

The Dbait molecules as disclosed herein must have at least one free end, as a mimic of double strand breaks (DSB). Said free end may be either a free blunt end or a 5'-/3'- protruding end. The "free end" refers herein to a nucleic acid molecule, in particular a double-stranded nucleic acid portion, having both a 5' end and a 3' end or having either a 3'end or a 5' end. Optionally, one of the 5' and 3' end can be used to conjugate the nucleic acid molecule or can be linked to a blocking group, for instance a or 3'-3'nucleotide linkage.

In a particular embodiment, they contain only one free end. Preferably, Dbait molecules are made of hairpin nucleic acids with a double-stranded DNA stem and a loop. The loop can be a nucleic acid, or other chemical groups known by skilled person or a mixture thereof. A nucleotide linker may include from 2 to 10 nucleotides, preferably, 3, 4 or 5 nucleotides. Non-nucleotide linkers non exhaustively include abasic nucleotide, polyether, polyamine, polyamide, peptide, carbohydrate, lipid, polyhydrocarbon, or other polymeric compounds (e. g. oligoethylene glycols such as those having between 2 and 10 ethylene glycol units, preferably 3, 4, 5, 6, 7 or 8 ethylene glycol units). A preferred linker is selected from the group consisting of hexaethyleneglycol, tetradeoxythymidylate (T4) and other linkers such as 1,19-bis(phospho)-8-hydraza-2-hydroxy-4-oxa-9-oxo-nonadecane and 2,19-bis(phosphor)-8-hydraza-1-hydroxy-4-oxa-9-oxo-nonadecane. Accordingly, in a particular embodiment, the Dbait molecules can be a hairpin molecule having a double stranded portion or stem comprising at least 16, 18, 20, 22, 24, 26, 28, 30 or 32 consecutive nucleotides of Dbait32 (SEQ ID NO: 1), Dbait32Ha (SEQ ID NO: 2), Dbait32Hb (SEQ ID NO: 3), Dbait32Hc (SEQ ID NO: 4) or Dbait32Hd (SEQ ID NO: 5) and a loop being a hexaethyleneglycol linker, a tetradeoxythymidylate linker (T4) 1,19-bis(phospho)-8-hydraza-2-hydroxy-4-oxa-9-oxo-nonadecane or 2,19-bis(phosphor)-8-hydraza-1-hydroxy-4-oxa-9-oxo-nonadecane. In a more particular embodiment, those Dbait molecules can have a double stranded portion consisting in 20, 22, 24, 26, 28, 30 or 32 consecutive nucleotides of Dbait32 (SEQ ID NO: 1), Dbait32Ha (SEQ ID NO: 2), Dbait32Hb (SEQ ID NO: 3), Dbait32Hc (SEQ ID NO: 4) or Dbait32Hd (SEQ ID NO: 5).

Dbait molecules preferably comprise a 2'-deoxynucleotide backbone, and optionally comprise one or several (2, 3, 4, 5 or 6) modified nucleotides and/or nucleobases other than adenine, cytosine, guanine and thymine. Accordingly, the Dbait molecules are essentially a DNA structure. In particular, the double-strand portion or stem of the Dbait molecules is made of deoxyribonucleotides.

Preferred Dbait molecules comprise one or several chemically modified nucleotide(s) or group(s) at the end of one or of each strand, in particular in order to protect them from degradation. In a particular preferred embodiment, the free end(s) of the Dbait molecules is(are) protected by one, two or three modified phosphodiester backbones at the end of one or of each strand. Preferred chemical groups, in particular the modified phosphodiester backbone, comprise phosphorothioates. Alternatively, preferred Dbait have 3'- 3' nucleotide linkage, or nucleotides with methylphosphonate backbone. Other modified backbones are well known in the art and comprise phosphoramidates, morpholino nucleic acid, 2'-0,4'-C methylene/ethylene bridged locked nucleic acid, peptide nucleic acid (PNA), and short chain alkyl, or cycloalkyl intersugar linkages or short chain heteroatomic or heterocyclic intrasugar linkages of variable length, or any modified nucleotides known by skilled person. In a first preferred embodiment, the Dbait molecules have the free end(s) protected by one, two or three modified phosphodiester backbones at the end of one or of each strand, more preferably by three modified phosphodiester backbones (in particular phosphorothioate or methylphosphonate) at least at the 3'end, but still more preferably at both 5' and 3' ends.

In a most preferred embodiment, the Dbait molecule is a hairpin nucleic acid molecule comprising a DNA double-stranded portion or stem of 32 bp (e.g., with a sequence selected from the group consisting of SEQ ID Nos 1-5, in particular SEQ ID No 4) and a loop linking the two strands of the DNA double-stranded portion or stem comprising or consisting of a linker selected from the group consisting of hexaethyleneglycol, tetradeoxythymidylate (T4) and 1,19-bis(phospho)-8-hydraza-2-hydroxy-4-oxa-9-oxo-nonadecane and 2,19-bis(phosphor)-8-hydraza-1-hydroxy-4-oxa-9-oxo-nonadecane, the free ends of the DNA double-stranded portion or stem (i.e. at the opposite of the loop) having three modified phosphodiester backbones (in particular phosphorothioate internucleotidic links).

Said nucleic acid molecules are made by chemical synthesis, semi-biosynthesis or biosynthesis, any method of amplification, followed by any extraction and preparation methods and any chemical modification. Linkers are provided so as to be incorporable by standard nucleic acid chemical synthesis. More preferably, nucleic acid molecules are manufactured by specially designed convergent synthesis: two complementary strands are prepared by standard nucleic acid chemical synthesis with the incorporation of appropriate linker precursor, after their purification, they are covalently coupled together.

Optionally, the nucleic acid molecules may be conjugated to molecules facilitating endocytosis or cellular uptake.

In particular, the molecules facilitating endocytosis or cellular uptake may be lipophilic molecules such as cholesterol, single or double chain fatty acids, or ligands which target cell receptor enabling receptor mediated endocytosis, such as folic acid and folate derivatives or transferrin (Goldstein et al. Ann. Rev. Cell Biol. 1985 1:1-39; Leamon & Lowe, Proc Natl Acad Sci USA. 1991, 88: 5572-5576.). The molecule may also be tocopherol, sugar such as galactose and mannose and their oligosaccharide, peptide such as RGD and bombesin and protein such as integrin. Fatty acids may be saturated or unsaturated and be in C₄-C₂₈, preferably in C₁₄-C₂₂, still more preferably being in C₁₈ such as oleic acid or stearic acid. In particular, fatty acids may be octadecyl or dioleoyl. Fatty acids may be found as double chain form linked with in appropriate linker such as a glycerol, a phosphatidylcholine or ethanolamine and the like or linked together by the linkers used to attach on the Dbait molecule. As used herein, the term "folate" is meant to refer to folate and folate derivatives, including pteroic acid derivatives and analogs. The analogs and derivatives of folic acid suitable for use in the present invention include, but are not limited to, antifolates, dihydrofolates, tetrahydrofolates, folinic acid, pteropolyglutamic acid, 1-deza, 3-deaza, 5-deaza, 8-deaza, 10-deaza, 1,5-deaza, 5,10 dideaza, 8,10-dideaza, and 5,8-dideaza folates, antifolates, and pteroic acid derivatives. Additional folate analogs are described in US2004/242582. Accordingly, the molecule facilitating endocytosis may be selected from the group consisting of single or double chain fatty acids, folates and cholesterol. More preferably, the molecule facilitating endocytosis is selected from the group consisting of dioleoyl, octadecyl, folic acid, and cholesterol. In a most preferred embodiment, the nucleic acid molecule is conjugated to a cholesterol.

The Dbait molecules facilitating endocytosis may conjugated to molecules facilitating endocytosis, preferably through a linker. Any linker known in the art may be used to attach the molecule facilitating endocytosis to Dbait molecules For instance, WO09/126933 provides a broad review of convenient linkers pages 38-45. The linker can be non-exhaustively, aliphatic chain, polyether, polyamine, polyamide, peptide, carbohydrate, lipid, polyhydrocarbon, or other polymeric compounds (e. g. oligoethylene glycols such as those having between 2 and 10 ethylene glycol units, preferably 3, 4, 5, 6, 7 or 8 ethylene glycol units, still more preferably 3 ethylene glycol units), as well as incorporating any bonds that may be break down by chemical or enzymatical way, such as a disulfide linkage, a protected disulfide linkage, an acid labile linkage (e.g., hydrazone linkage), an ester linkage, an ortho ester linkage, a phosphonamide linkage, a biocleavable peptide linkage, an azo linkage or an aldehyde linkage. Such cleavable linkers are detailed in WO2007/040469 pages 12-14, in WO2008/022309 pages 22-28.

In a particular embodiment, the nucleic acid molecule can be linked to one molecule facilitating endocytosis. Alternatively, several molecules facilitating endocytosis (e.g., two, three or four) can be attached to one nucleic acid molecule.

In a specific embodiment, the linker between the molecule facilitating endocytosis, in particular cholesterol, and nucleic acid molecule is CO-NH-(CH₂-CH₂-O)ₙ, wherein n is an integer from 1 to 10, preferably n being selected from the group consisting of 3, 4, 5 and 6. In a very particular embodiment, the linker is CO-NH-(CH₂-CH₂-O)₄ (carboxamido tetraethylene glycol) or CO-NH-(CH₂-CH₂-O)₃ (carboxamido triethylene glycol). The linker can be linked to nucleic acid molecules at any convenient position which does not modify the activity of the nucleic acid molecules. In particular, the linker can be linked at the 5' end. Therefore, in a preferred embodiment, the contemplated conjugated Dbait molecule is a Dbait molecule having a hairpin structure and being conjugated to the molecule facilitating endocytosis, preferably through a linker, at its 5' end.

In another specific embodiment, the linker between the molecule facilitating endocytosis, in particular cholesterol, and nucleic acid molecule is dialkyl-disulfide {e.g., (CH₂)ᵣ-S-S-(CH₂)ₛ with r and s being integer from 1 to 10, preferably from 3 to 8, for instance 6}.

In a most preferred embodiment, the conjugated Dbait molecule is a hairpin nucleic acid molecule comprising a DNA double-stranded portion or stem of 32 bp and a loop linking the two strands of the DNA double-stranded portion or stem comprising or consisting of a linker selected from the group consisting of hexaethyleneglycol, tetradeoxythymidylate (T4), 1,19-bis(phospho)-8-hydraza-2-hydroxy-4-oxa-9-oxo-nonadecane and 2,19-bis(phosphor)-8-hydraza-1-hydroxy-4-oxa-9-oxo-nonadecane, the free ends of the DNA double-stranded portion or stem (i.e. at the opposite of the loop) having three modified phosphodiester backbones (in particular phosphorothioate internucleotidic links) and said Dbait molecule being conjugated to a cholesterol at its 5' end, preferably through a linker (e.g. carboxamido oligoethylene glycol, preferably carboxamido triethylene or tetraethylene glycol).

In a particular embodiment, the Dbait molecules can be conjugated Dbait molecules such as those extensively described in PCT patent application WO2011/161075, the disclosure of which is incorporated herein by reference.

In a preferred embodiment, NNNN-(N)ₙ-N comprises at least 6, 8, 10, 12, 14, 16, 18, 20, 22, 24, 26, 28, 30 or 32 consecutive nucleotides of Dbait32 (SEQ ID NO: 1), Dbait32Ha (SEQ ID NO: 2), Dbait32Hb (SEQ ID NO: 3), Dbait32Hc (SEQ ID NO: 4) or Dbait32Hd (SEQ ID NO: 5) or consists in 20, 22, 24, 26, 28, 30 or 32 consecutive nucleotides of Dbait32, Dbait32Ha, Dbait32Hb, Dbait32Hc or Dbait32Hd. In a particular embodiment, NNNN-(N)ₙ-N comprises or consists in Dbait32 (SEQ ID NO: 1), Dbait32Ha (SEQ ID NO: 2), Dbait32Hb (SEQ ID NO: 3), Dbait32Hc (SEQ ID NO: 4) or Dbait32Hd (SEQ ID NO: 5), more preferably Dbait32Hc (SEQ ID NO: 4).

According, the conjugated Dbait molecules may be selected from the group consisting of:
with NNNN-(N)ₙ-N being SEQ ID NO: 1;
with NNNN-(N)ₙ-N being SEQ ID NO: 2;
with NNNN-(N)ₙ-N being SEQ ID NO: 3;
with NNNN-(N)ₙ-N being SEQ ID NO: 4; or
with NNNN-(N)ₙ-N being SEQ ID NO: 5

In one preferred embodiment, the Dbait molecule has the following formula: 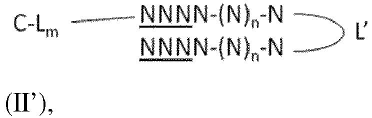 wherein
- NNNN-(N)ₙ-N comprises 28, 30 or 32 nucleotides , preferably 32 nucleotides and/or
- the underlined nucleotide refers to a nucleotide having or not a phosphorothioate or methylphosphonate backbone, more preferably a phosphorothioate backbone; preferably, the underlined nucleotide refers to a nucleotide having a phosphorothioate or methylphosphonate backbone, more preferably a phosphorothioate backbone and/or,
- the linker L' is selected from the group consisting of hexaethyleneglycol, tetradeoxythymidylate (T4), 1,19-bis(phospho)-8-hydraza-2-hydroxy-4-oxa-9-oxo-nonadecane or 2,19-bis(phosphor)-8-hydraza-1-hydroxy-4-oxa-9-oxo-nonadecane; and/or,
- m is 1 and L is a carboxamido polyethylene glycol, more preferably carboxamido triethylene or tetraethylene glycol; and/or,
- p is 1; and/or,
- C is selected from the group consisting of a cholesterol, single or double chain fatty acids such as octadecyl, oleic acid, dioleoyl or stearic acid, or ligand (including peptide, protein, aptamer) which targets cell receptor such as folic acid, tocopherol, sugar such as galactose and mannose and their oligosaccharide, peptide such as RGD and bombesin, and protein such transferring and integrin, preferably is a cholesterol.

In a very specific embodiment, the Dbait molecule (also referred herein as AsiDNA) has the following formula: 
C-L_m GCTGTGCCCACAACCCAGCAAACAAGCCTAGA L'
CGACACGGGTGTTGGGTCGTTTGTTCGGATCT (IIa) (SEQ ID NO: 6)
 wherein C-Lₘ is the tetraethyleneglycol linker (10-O-[1-propyl-3-N-carbamoylcholesteryl]-tetraethyleneglycol radical, and L' is 1,19-bis(phospho)-8-hydraza-2-hydroxy-4-oxa-9-oxo-nonadecane; also represented by the following formula:

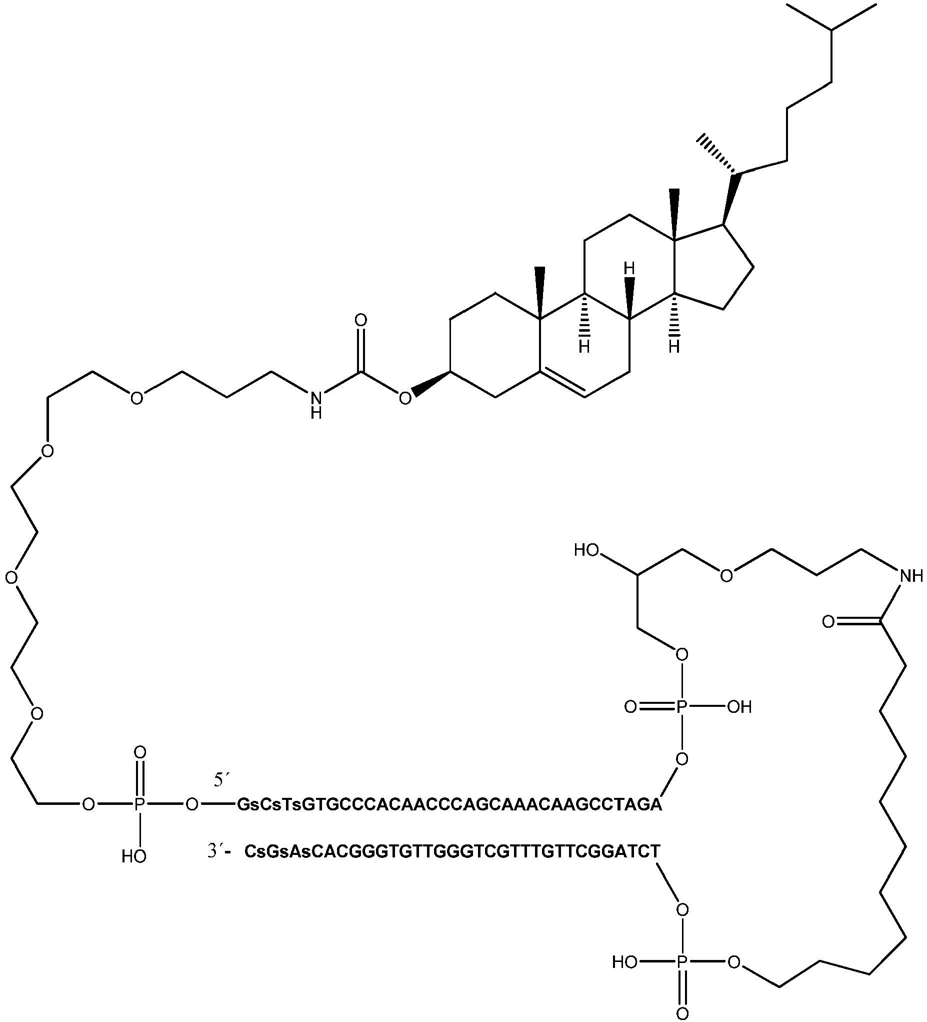

### Cancers or tumors to be treated:

The terms "cancer", "cancerous", or "malignant" refer to or describe the physiological condition in mammals that is typically characterized by unregulated cell growth. Examples of cancer include, for example, leukemia, lymphoma, blastoma, carcinoma and sarcoma.

Various cancers are also encompassed by the scope of the invention, including, but not limited to, the following: carcinoma including that of the bladder (including accelerated and metastatic bladder cancer), breast, colon (including colorectal cancer), kidney, liver, lung (including small and non-small cell lung cancer and lung adenocarcinoma), ovary, prostate, testis, genitourinary tract, lymphatic system, rectum, larynx, pancreas (including exocrine pancreatic carcinoma), esophagus, stomach, gall bladder, cervix, thyroid, and skin (including squamous cell carcinoma); hematopoietic tumors of lymphoid lineage including leukemia, acute lymphocytic leukemia, acute lymphoblastic leukemia, B-cell lymphoma, T-cell lymphoma (including cutaneous or peripheral T-cell lymphoma), Hodgkins lymphoma, non-Hodgkins lymphoma, hairy cell lymphoma, histiocytic lymphoma, and Burketts lymphoma; hematopoietic tumors of myeloid lineage including acute and chronic myelogenous leukemias, myelodysplastic syndrome, myeloid leukemia, and promyelocytic leukemia; tumors of the central and peripheral nervous system including astrocytoma, neuroblastoma, glioma, and schwannomas; tumors of mesenchymal origin including fibrosarcoma, rhabdomyosarcoma, and osteosarcoma; other tumors including melanoma, xenoderma pigmentosum, keratoactanthoma, seminoma, thyroid follicular cancer, and teratocarcinoma; melanoma, unresectable stage III or IV malignant melanoma, squamous cell carcinoma, small-cell lung cancer, non-small cell lung cancer, glioma, gastrointestinal cancer, renal cancer, ovarian cancer, liver cancer, colorectal cancer, endometrial cancer, kidney cancer, prostate cancer, thyroid cancer, neuroblastoma, pancreatic cancer, glioblastoma multiforme, cervical cancer, stomach cancer, bladder cancer, hepatocarcinoma, breast cancer, colon carcinoma, and head and neck cancer, retinoblastoma, gastric cancer, germ cell tumor, bone cancer, bone tumors, adult malignant fibrous histiocytoma of bone; childhood malignant fibrous histiocytoma of bone, sarcoma, pediatric sarcoma; myelodysplastic syndromes; neuroblastoma; testicular germ cell tumor, intraocular melanoma, myelodysplastic syndromes; myelodysplastic/myeloproliferative diseases, synovial sarcoma.

In a preferred embodiment of the present invention, the cancer is a solid tumor. For instance, the cancer may be sarcoma and osteosarcoma such as Kaposi sarcome, AIDS-related Kaposi sarcoma, melanoma, in particular uveal melanoma, and cancers of the head and neck, kidney, ovary, pancreas, prostate, thyroid, lung, esophagus, breast in particular triple negative breast cancer (TNBC), bladder, colorectum, liver and biliary tract, uterine, appendix, and cervix, testicular cancer, gastrointestinal cancers and endometrial and peritoneal cancers. Preferably, the cancer may be sarcoma, melanoma, in particular uveal melanoma, and cancers of the head and neck, kidney, ovary, pancreas, prostate, thyroid, lung, esophagus, breast in particular (TNBC), bladder, colorectum, liver, cervix, and endometrial and peritoneal cancers.

The pharmaceutical compositions and the products, kits or combined preparations described in the invention may be useful for inhibiting the growth of solid tumors, decreasing the tumor volume, preventing the metastatic spread of tumors and the growth or development of micrometastases, preventing the tumor recurrence and preventing the tumor relapse. The pharmaceutical compositions and the products, kits or combined preparations described in the invention are in particular suitable for the treatment of poor prognosis patients or of radio- or chemo-resistant tumors. In a particular embodiment, the cancer is a high-grade or advanced cancer or is a metastatic cancer.

### Regimen, dosages and administration routes:

The effective dosage of each of the combination partners employed in the combined preparation of the invention may vary depending on the particular compound or pharmaceutical composition employed, the mode of administration, the condition being treated, the severity of the condition being treated. Thus, the dosage regimen of the combined preparation of the invention is selected in accordance with a variety of factors including the route of administration and the patient status. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites.

The invention more particularly relates to a pharmaceutical composition, a kit, product or combined preparation wherein the amount or dosage of the HDAC inhibitor can be lowered in comparison with its amount or dosage when it is used alone. Indeed, the combination of a Dbait molecule and a HDAC inhibitor leads to a clear synergistic effect of the two active ingredients. This potentiating effect allows the decrease of the amount of the HDAC inhibitor, which generally exhibit a toxicity for the normal cells and therefore can be associated with adverse effects. The Dbait molecules advantageously exhibit a minimal toxicity, and even no toxicity. Then, with the combined treatment of the invention, it is possible to preserve the efficacy of the treatment, or even to improve it, while decreasing its adverse effects, in particular the adverse effects of the HDAC inhibitor.

Alternatively, instead of lowering the amount or dosage of the HDAC inhibitor, the administration frequency of the HDAC inhibitor or its or treatment period can be reduced.

The invention also relates to a method for treating cancer, to a pharmaceutical composition, to a product, kit or combined preparation as disclosed above, wherein the amounts of the Dbait molecule and the HDAC inhibitor in the combined preparation are such that the combined therapeutic effect of the two active ingredients is synergistic.

By the term "synergistic" therapeutic effect is meant that the obtained therapeutic effect of the combination is more than the addition of the therapeutic effect of each partner alone (i.e. more than the effect of the Dbait molecule alone plus the effect of the HDAC inhibitor alone).

The invention relates to a method for the treatment of a cancer, to a pharmaceutical composition, to a product, kit or combined preparation as disclosed above, wherein the HDAC inhibitor can be used at the same dosage or at lower dosage than the conventional dosage used in chemotherapy for the same indication and the same administration route when it is used alone (i.e., an amount equal to or preferably lower than the one used in conventional chemotherapy), also called herein a sub-therapeutic amount.

More particularly, the amount can be for instance 100, 90, 80, 70, 60, 50, 40, 30, 20 or 10 % of the conventional therapeutic dosage (in particular for the same indication and the same administration route). The conventional therapeutic dosages are those acknowledged by the drug approvals agencies (e.g., FDA or EMEA). In that respect, the present invention relates to a method for the treatment of a cancer, to a pharmaceutical composition, to a product, kit or combined preparation as disclosed above, wherein the amount of the HDAC inhibitor is used at a sub-therapeutic dosage and the amount of Dbait molecule as disclosed herein is such that the combined therapeutic effect of the two active ingredients is synergistic.

Determining a synergistic interaction between two components, the optimum range for the effect and absolute dose ranges of each component for the effect may be definitively measured by administration of the components over different w/w ratio ranges and doses to patients in need of treatment. For humans, the complexity and cost of carrying out clinical studies on patients may render impractical the use of this form of testing as a primary model for synergy. However, the observation of synergy in one species can be predictive of the effect in other species and animal models exist to measure a synergistic effect and the results of such studies can also be used to predict effective dose and plasma concentration ratio ranges and the absolute doses and plasma concentrations required in other species by the application of pharmacokinetic/pharmacodynamic methods. Correlations between cancer models and effects seen in man suggest that observed synergy on animal models may be predictive of a synergy on man too.

The pharmacological activity of a combination of the invention may, for example, be demonstrated in a clinical study or more preferably in a test procedure. Suitable clinical studies are, for example, open label non-randomized, dose escalation studies in patients with advanced tumors. Such studies can prove the synergism of the active ingredients of the combination of the invention. The beneficial effects on proliferative diseases can be determined directly through the results of these studies or by changes in the study design which are known as such to a person skilled in the art. Such studies are, in particular, suitable to compare the effects of a monotherapy using the active ingredients and a combination of the invention. Preferably, the combination partner (a) is administered with a fixed dose and the dose of the combination partner (b) is escalated until the maximum tolerated dosage is reached. Alternatively, the combination partner (b) is administered with a fixed dose and the dose of the combination partner (a) is escalated until the maximum tolerated dosage is reached.

The treatment may include one or several cycles, for instance two to ten cycles, in particular two, three, four or five cycles. The cycles may be continued or separated. For instance, each cycle is separated by a period of time of one to eight weeks, preferably three to four weeks.

### Biomarkers:

In some embodiments, one or more biomarkers may be used to monitor or determine the efficacy of the treatment. In certain embodiments, the percentage of cancer cells with micronuclei (MN) in paired samples may be used as a biomarker; micronuclei is believed to correlate with the level of the inhibition of HDAC activity. For instance, in some embodiments, the percentage of cancer cells with micronuclei in a tumor biopsy may be used to determine the efficacy of monotherapy with an HDACi inhibitor (such as belinostat) or combination therapy including a Dbait molecule in a subject. In some such embodiments, a first tumor biopsy may be taken between about one to about three hours (e.g., two hours) before administration of the HDAC inhibitor and a second tumor biopsy may be taken about several hours (e.g., twelve hours) after administration of the HDAC inhibitor. The percentage of cancer cells with micronuclei in the first biopsy is set to be 100%, such that the percentage of cancer cells with micronuclei in the second biopsy is normalized by the amount in the first biopsy.

Criteria for identifying micronuclei are well-established, for instance as described in Countryman & Heddle (1976, Mutation Research, 41, 321-332). In particular, the criteria are the followings:
- diameter less than 1/3rd the main nucleus;
- non-refractility (to exclude small stain particles);
- colour same as or lighter than the nucleus (to exclude large stain particles);
- location within 3 or 4 nuclear diameters of of a nucleus; and not touching the nucleus (to make frequency measurements meaningful); and
- no more than 2 micronuclei associated with one nucleus.

The counting of the number of cancer cells having micronuclei can be determined under microscope after appropriate staining. The micronuclei are clearly smaller than the cell nuclei and are physically separate from them. The counting can be carried out by visual scoring or by automated scoring procedures. Prototypes of software for automated image analysis have been described in the literature since the early 1990s and developed by Becton-Dickinson and Loats Associates, Inc (LAI Automated Micronucleus Assay System;. 201 East Main St. Westminster, MD 21157 410-876-8055).

In one embodiment, the method for determining the frequency of cancer cells with micronuclei comprises lysing cells of the sample to release nuclei and micronuclei, counting the nuclei and micronuclei, and determining the frequency of cancer cells with micronuclei. The lysis can be achieved by any known procedures, such as those described in Nusse and Marx (1997, Mutat Res. 392(1-2):109-15), the disclosure of which being incorporated by reference. The nuclei and micronuclei can be analyzed by flow cytometry. When nuclei and micronuclei are analyzed by flow cytometry, the nuclei and micronuclei are stained with a dye prior to flow cytometry.

Analysis of nuclei and micronuclei can be performed by flow cytometry as described in Nusse and Marx (1997, Mutat Res. 392(1-2):109-15), the disclosure of which being incorporated by reference. In this embodiment, nuclei may be distinguished from micronuclei by their greater light scatter and greater DNA content. The frequency of cells with micronuclei is calculated as the micronuclei frequency per nuclei.

The micronuclei and nuclei can be stained by any appropriate dye. In a preferred embodiment, the dye is a fluorescent dye. For instance, the florescent dye can be selected from the group consisting of DAPI (4',6-diamidino-2-phenylindole), propidium iodide, Hoechst 33342, carboxyfluorescein diacetate succinimidyl ester (CFSE) and dyes of the PKH series. When nuclei and micronuclei are analyzed by flow cytometry, the dye can be selected from the group consisting of DAPI (4',6-diamidino-2-phenylindole), propidium iodide and Hoechst 33342. It will be appreciated by those skilled in the art that a number of alternative nucleic acid dyes may also be used.

For instance, a method for determining the frequency of micronuclei can comprise:
- incubating cells from the sample for a sufficient time to allow a substantial population of cells to complete at least one cellular division;
- optionally, sorting the cells to obtain a substantially pure population of cells which have undergone one cellular division;
- lysing the cells to release nuclei and micronuclei; and
- determining the nuclei and micronuclei in order to determine the frequency of micronuclei.

Based on the frequency of cancer cells with micronuclei, it is possible to monitor or determine the efficacy of the treatment of the tumor to a treatment with HDACi monotherapy or with combination therapy of the invention. An increase in the frequency of cancer cells with micronuclei between the first tumor biopsy and the second tumor biopsy is indicative that the treatment with HDACi monotherapy or with combination therapy of the invention is effective.

In other embodiments, the level of gamma-H2AX in paired samples may be used as a biomarker; gamma H2AX is believed to correlate with the level of the inhibition of HDAC activity. For instance, in some embodiments, the level of gamma-H2AX in a tumor biopsy may be used to determine the efficacy of monotherapy with an HDACi inhibitor (such as belinostat) or combination therapy including a Dbait molecule in a subject. In some such embodiments, a first tumor biopsy may be taken between about one to about three hours (e.g., two hours) before administration of the HDAC inhibitor and a second tumor biopsy may be taken about several hours (e.g., twelve hours) after administration of the HDAC inhibitor. The level of gamma-H2AX in cancer cells in the first biopsy is set to be 100%, such that level of gamma-H2AX in the second biopsy is normalized by the amount in the first biopsy.

In one embodiment, the method for determining the level of gamma-H2AX may be performed by using an antibody raised against gamma-H2AX which can therefore be visualized by immunofluorescence through secondary antibodies. The detection and visualization of gamma-H2AX by flow cytometry allow to monitor or determine the efficacy of the treatment of the tumor with HDACi monotherapy or with combination therapy of the invention. An increase in the level of gamma-H2AX between the first tumor biopsy and the second tumor biopsy is indicative that the treatment with HDACi monotherapy or with combination therapy of the invention is effective.

Further aspects and advantages of the present invention will be described in the following examples, which should be regarded as illustrative and not limiting.

### Examples

### EXAMPLE 1: Efficacy of the combined treatment AsiDNA and belinostat

### Materials and Methods

### Cell culture

Cell cultures were performed with the triple negative breast cancer cell line MDA-MB-231, the histiocytic lymphoma cell line U937 and the non-tumor mammary cell line MCF-10A. Cells were grown according to the supplier's instructions. Cell lines were maintained at 37°C in a humidified atmosphere at 5% CO₂, except the MDA-MB-231 cell line which was maintained at 0% CO₂.

### Drugs treatment, measurement of cellular survival and synergy analysis

In a first step, the characterization of the effect of drugs monotherapy on cell survival (tumour and non-tumour cells) was performed. MDA-MB-231 (5.10³ cells/well), MCF-10A (5.10³ cells/well) and U937 (2.10⁴ cells/well) were seeded in 96 well-plates and incubated 24 hours at +37°C before drug addition with increasing concentrations for 4 to 7 days. Following drug exposure, cell survival was measured using the XTT assay (Sigma Aldrich). Briefly, the XTT solution was added directly to each well containing cell culture and the cells incubated for 4 to 24 hours at 37°C before reading the absorbance at 490 nm and 690 nm using a microplate reader (BMG Fluostar, Galaxy). Cell survival was calculated as the ratio of living treated cells to living mock-treated cells. The IC50 (which represents the dose at which 50% of the cells are viable) was calculated by a non-linear regression model using GraphPad Prism software (version 5.04) by plotting the percentage viability against the Log of the drug concentration on each cell line.

In a second step, analysis and quantification of the drug combination efficacy were performed. Cells were seeded in 96-well plates at the appropriate densities and treated with AsiDNA and/or the HDAC inhibitor (HDACi) belinostat simultaneously during 4 to 7days. AsiDNA and belinostat combinations were performed at a constant ratio (from IC50/8 to IC50*8) as previously described (Chou TC, Talalay P. Adv Enzyme Regul. 1984;22:27-55 (Quantitative analysis of dose-effect relationships: the combined effects of multiple drugs or enzyme inhibitors). Cell survival was measured using the XTT assay (see above).

Combination efficacy was evaluated by "the combination index (CI)" which was determined using the CalcuSyn software tool based on the Chou Talalay method). A CI could be interpreted as follows: CI<1, =1, and >1 indicate synergism; additive effect and antagonism, respectively (CalculSyn manual, Biosoft, 2006). Note that we define synergism as a more than expected additive effect, and antagonism as a less than expected additive effect.

### Statistical analysis

All statistical analysis were performed with a two-tailed Student *t* test.

### Results

The inventors tested the effect of the combination of AsiDNA with the HDAC inhibitor belinostat on the survival of cancer cells as well as on normal cells (non-cancer cells).

More particularly, the results are shown in **Figure 1****.**

Tumor cells (MDA-MB-231, U937) were treated with AsiDNA (light grey), belinostat (dark grey) or both (black) at doses corresponding to the IC50 (**Table 1**). The same drug doses as MDA-MB-231 were applied to MCF-10A non-tumor cells. Survival was measured 4 days (U937) or 7 days (MDA-MB-231 and MCF-10A) after treatment using the XTT assay. Results are presented as % to non-treated cells. In tumor cells, where monotherapy with AsiDNA or belinostat resulted in a cell survival of 40 to 60% (compared to non-treated cells), combined treatments AsiDNA+belinostat decreased significantly cell survival (**Figure 1A**). The non-tumor cells MCF-10A were insensitive to monotherapies as well as combined therapies. Synergy analysis revealed a synergistic efficacy of the combined treatment AsiDNA+belinostat on MDA-MB-231 and U937 cell lines (CI<1), compared to non-tumor cells (**Figure 1B**). Interestingly, a high synergy between AsiDNA and Belinostat was observed even at very low doses, making the combination very attractive for a clinical investigation (data not shown or figure). Absence of any effect in non-tumor cells predicts a non-toxicity and a high safety of this combined treatment in normal tissues.

**Table 1: Drug half maximal inhibitory concentrations (IC50) in tumor cells.**

| IC50 were calculated according to the dose-response curves of the different inhibitors using GraphPadPrism software. | | | | | |
|---|---|---|---|---|---|
| | **IC50** (**µM**) | | | | |
| | **AsiDNA** | **Belinostat** | **Vorinostat** | **Entinostat** | **Romidepsin** |
| **MDA-MB-231** | 12.5 | 0.25 | 1.5 | 0,1 | 0,0004 |
| **U937** | 2.5 | 0.25 | 0.6 | 0,01 | 0,001 |

### EXAMPLE 2: Efficacy of the combined treatment AsiDNA and Vorinostat

### Materials and Methods

### Cell culture

Cell cultures were performed with the triple negative breast cancer cell line MDA-MB-231, the histiocytic lymphoma cell line U937 and the non-tumor mammary cell line MCF-10A. Cells were grown according to the supplier's instructions. Cell lines were maintained at 37°C in a humidified atmosphere at 5% CO₂, except the MDA-MB-231 cell line which was maintained at 0% CO₂.

### Drugs treatment, measurement of cellular survival and synergy analysis

In a first step, the characterization of the effect of drugs monotherapy on cell survival (tumour and non-tumour cells) was performed. MDA-MB-231 (5.10³ cells/well), MCF-10A (5.10³ cells/well) and U937 (2.10⁴ cells/well) were seeded in 96 well-plates and incubated 24 hours at +37°C before drug addition with increasing concentrations for 4 to 7 days. Following drug exposure, cell survival was measured using the XTT assay (Sigma Aldrich). Briefly, the XTT solution was added directly to each well containing cell culture and the cells incubated for 4 to 24 hours at 37°C before reading the absorbance at 490 nm and 690 nm using a microplate reader (BMG Fluostar, Galaxy). Cell survival was calculated as the ratio of living treated cells to living mock-treated cells. The IC50 (which represents the dose at which 50% of the cells are viable) was calculated by a non-linear regression model using GraphPad Prism software (version 5.04) by plotting the percentage viability against the Log of the drug concentration on each cell line. In a second step, analysis and quantification of the drug combination efficacy were performed. Cells were seeded in 96-well plates at the appropriate densities and treated with AsiDNA and/or the HDAC inhibitor (HDACi) vorinostat simultaneously during 4 to 7days. AsiDNA and vorinostat combinations were performed at a constant ratio (from IC50/8 to IC50*8) as previously described. Cell survival was measured using the XTT assay (see above). Combination efficacy was evaluated by "the combination index (CI)" which was determined using the CalcuSyn software tool based on the Chou Talalay method. A CI could be interpreted as follows: CI<1, =1, and >1 indicate synergism; additive effect and antagonism, respectively (CalculSyn manual, Biosoft, 2006). Note that we define synergism as a more than expected additive effect, and antagonism as a less than expected additive effect.

### Statistical analysis

All statistical analysis were performed with a two-tailed Student *t* test.

### Results

The inventors tested the effect of the combination of AsiDNA with the HDAC inhibitor vorinostat on the survival of cancer cells as well as on normal cells (non-cancer cells).

More particularly, the results are shown in **Figure 2****.**

Tumor cells (MDA-MB-231, U937) were treated with AsiDNA (light grey), vorinostat (dark grey) or both (black) at doses corresponding to the IC50 (**Table 1**). The same drug doses as MDA-MB-231 were applied to MCF-10A non-tumor cells. Survival was measured 4 days (U937) or 7 days (MDA-MB-231 and MCF-10A) after treatment using the XTT assay. Results are presented as % to non-treated cells. In tumor cells, where monotherapy with AsiDNA or vorinostat resulted in a cell survival of 30 to 80% (compared to non-treated cells), combined treatments AsiDNA+ vorinostat decreased significantly cell survival (**Figure 2A**). The non-tumor cells MCF-10A were insensitive to AsiDNA. However, they showed a sensitivity to the HDACi vorinostat. Adding AsiDNA didn't increase the sensitivity of non-tumor cells to romidepsin. Synergy analysis revealed a synergistic efficacy of the combined treatment AsiDNA+ vorinostat on MDA-MB-231 and U937 cell lines (CI<1), compared to non-tumor cells (**Figure 2B**).

### EXAMPLE 3: Efficacy of the combined treatment AsiDNA and Entinostat

### Materials and Methods

### Cell culture

Cell cultures were performed with the triple negative breast cancer cell line MDA-MB-231, the histiocytic lymphoma cell line U937 and the non-tumor mammary cell line MCF-10A. Cells were grown according to the supplier's instructions. Cell lines were maintained at 37°C in a humidified atmosphere at 5% CO₂, except the MDA-MB-231 cell line which was maintained at 0% CO₂.

### Drugs treatment, measurement of cellular survival and synergy analysis

In a first step, characterization of the effect of drugs monotherapy on cell survival (tumour and non-tumour cells) was performed. MDA-MB-231 (5.10³ cells/well), MCF-10A (5.10³ cells/well) and U937 (2.10⁴ cells/well) were seeded in 96 well-plates and incubated 24 hours at +37°C before drug addition with increasing concentrations for 4 to 7 days. Following drug exposure, cell survival was measured using the XTT assay (Sigma Aldrich). Briefly, the XTT solution was added directly to each well containing cell culture and the cells incubated for 4 to 24 hours at 37°C before reading the absorbance at 490 nm and 690 nm using a microplate reader (BMG Fluostar, Galaxy). Cell survival was calculated as the ratio of living treated cells to living mock-treated cells. The IC50 (which represents the dose at which 50% of the cells are viable) was calculated by a non-linear regression model using GraphPad Prism software (version 5.04) by plotting the percentage viability against the Log of the drug concentration on each cell line. In a second step, analysis and quantification of the drug combination efficacy were performed. Cells were seeded in 96-well plates at the appropriate densities and treated with AsiDNA and/or the HDAC inhibitor (HDACi) entinostat simultaneously during 4 to 7days. AsiDNA and entinostat combinations were performed at a constant ratio (from IC50/8 to IC50*8) as previously described. Cell survival was measured using the XTT assay (see above). Combination efficacy was evaluated by "the combination index (CI)" which was determined using the CalcuSyn software tool based on the Chou Talalay method. A CI could be interpreted as follows: CI<1, =1, and >1 indicate synergism; additive effect and antagonism, respectively (CalculSyn manual, Biosoft, 2006). Note that we define synergism as a more than expected additive effect, and antagonism as a less than expected additive effect.

### Statistical analysis

All statistical analysis were performed with a two-tailed Student *t* test.

### Results

The inventors tested the effect of the combination of AsiDNA with the HDAC inhibitor entinostat on the survival of cancer cells as well as on normal cells (non-cancer cells).

More particularly, the results are shown in **Figure 3****.**

Tumor cells (MDA-MB-231, U937) were treated with AsiDNA (light grey), entinostat (dark grey) or both (black) at doses corresponding to the IC50 (**Table 1**). The same drug doses as MDA-MB-231 were applied to MCF-10A non-tumor cells. Survival was measured 4 days (U937) or 7 days (MDA-MB-231 and MCF-10A) after treatment using the XTT assay. Results are presented as % to non-treated cells. In tumor cells, where monotherapy with AsiDNA or entinostat resulted in a cell survival of 30 to 70% (compared to non-treated cells), combined treatments AsiDNA+ entinostat decreased significantly cell survival (**Figure 3A**). The non-tumor cells MCF-10A were insensitive to monotherapies as well as combined therapies. Synergy analysis revealed a synergistic efficacy of the combined treatment AsiDNA+ entinostat on MDA-MB-231 and U937 cell lines (CI<1), compared to non-tumor cells (**Figure 3B**).

### EXAMPLE 4: Efficacy of the combined treatment AsiDNA and romidepsin

### Materials and Methods

### Cell culture

Cell cultures were performed with the acute lymphoblastic leukemia cell line MOLT-4, the histiocytic lymphoma cell line U937 and the non-tumor mammary cell line MCF-10A. Cells were grown according to the supplier's instructions. Cell lines were maintained at 37°C in a humidified atmosphere at 5% CO₂, except the MDA-MB-231 cell line which was maintained at 0% CO₂.

### Drugs treatment, measurement of cellular survival and synergy analysis

In a first step, the characterization of the effect of drugs monotherapy on cell survival (tumour and non-tumour cells) was performed. MDA-MB-231 (5.10³ cells/well), MCF-10A (5.10³ cells/well) and U937 (2.10⁴ cells/well) were seeded in 96 well-plates and incubated 24 hours at +37°C before drug addition with increasing concentrations for 4 to 7 days. Following drug exposure, cell survival was measured using the XTT assay (Sigma Aldrich). Briefly, the XTT solution was added directly to each well containing cell culture and the cells incubated for 4 to 24 hours at 37°C before reading the absorbance at 490 nm and 690 nm using a microplate reader (BMG Fluostar, Galaxy). Cell survival was calculated as the ratio of living treated cells to living mock-treated cells. The IC50 (which represents the dose at which 50% of the cells are viable) was calculated by a non-linear regression model using GraphPad Prism software (version 5.04) by plotting the percentage viability against the Log of the drug concentration on each cell line. In a second step, analysis and quantification of the drug combination efficacy were performed. Cells were seeded in 96-well plates at the appropriate densities and treated with AsiDNA and/or the HDAC inhibitor (HDACi) romidepsin simultaneously during 4 to 7days. AsiDNA and romidepsin combinations were performed at a constant ratio (from IC50/8 to IC50*8) as previously described¹. Cell survival was measured using the XTT assay (see above). Combination efficacy was evaluated by "the combination index (CI)" which was determined using the CalcuSyn software tool based on the Chou Talalay method¹. A CI could be interpreted as follows: CI<1, =1, and >1 indicate synergism; additive effect and antagonism, respectively (CalculSyn manual, Biosoft, 2006). Note that we define synergism as a more than expected additive effect, and antagonism as a less than expected additive effect.

### Statistical analysis

All statistical analysis were performed with a two-tailed Student *t* test.

### Results

The inventors tested the effect of the combination of AsiDNA with the HDAC inhibitor romidepsin on the survival of cancer cells as well as on normal cells (non-cancer cells). More particularly, the results are shown in **Figure 4****.**

Tumor cells (MOLT-4, U937) were treated with AsiDNA (light grey), romidepsin (dark grey) or both (black) at doses corresponding to the IC50 (**Table 1**). Survival was measured 4 days (MOLT-4 and U937) or 7 days (MCF-10A) after treatment using the XTT assay. Results are presented as % to non-treated cells. In tumor cells, where monotherapy with AsiDNA or romidepsin resulted in a cell survival of 30 to 70% (compared to non-treated cells), combined treatments AsiDNA+ romidepsin decreased significantly cell survival (**Figure 4A**). The non-tumor cells MCF-10A were insensitive to AsiDNA. However, they showed a sensitivity to the HDACi romidepsin. Adding AsiDNA didn't increase the sensitivity of non-tumor cells to romidepsin. Synergy analysis revealed a synergistic efficacy of the combined treatment AsiDNA + romidepsin on MOLT-4 and U937 cell lines (CI<1), compared to non-tumor cells (**Figure 4B**).

## Claims

1. A pharmaceutical composition or a kit (kit-of-parts) comprising a Dbait molecule and a HDAC inhibitor.

2. The pharmaceutical composition or the kit (kit-of-parts) according to claim 1, wherein the Dbait molecule has one of the following formulae: 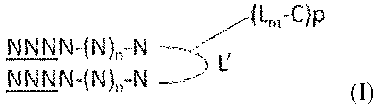 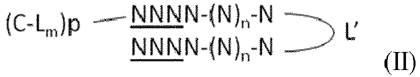 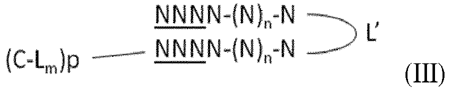 wherein N is a deoxynucleotide, n is an integer from 1 to 195, the underlined N refers to a nucleotide having or not a modified phosphodiester backbone, L' is a linker, C is the molecule facilitating endocytosis selected from a lipophilic molecule or a ligand which targets cell receptor enabling receptor mediated endocytosis, L is a linker, m and p, independently, are an integer being 0 or 1.

3. The pharmaceutical composition or the kit (kit-of-parts) according to claim 1 or 2, wherein the Dbait molecule has the following formula: 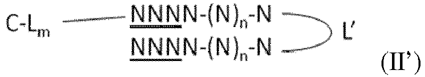 with the same definition than formulae (I), (II), and (III) for N, N, n, L, L', C and m.

4. The pharmaceutical composition or the kit (kit-of-parts) according to anyone claims 1 to 3, wherein the Dbait molecule has the following formula:

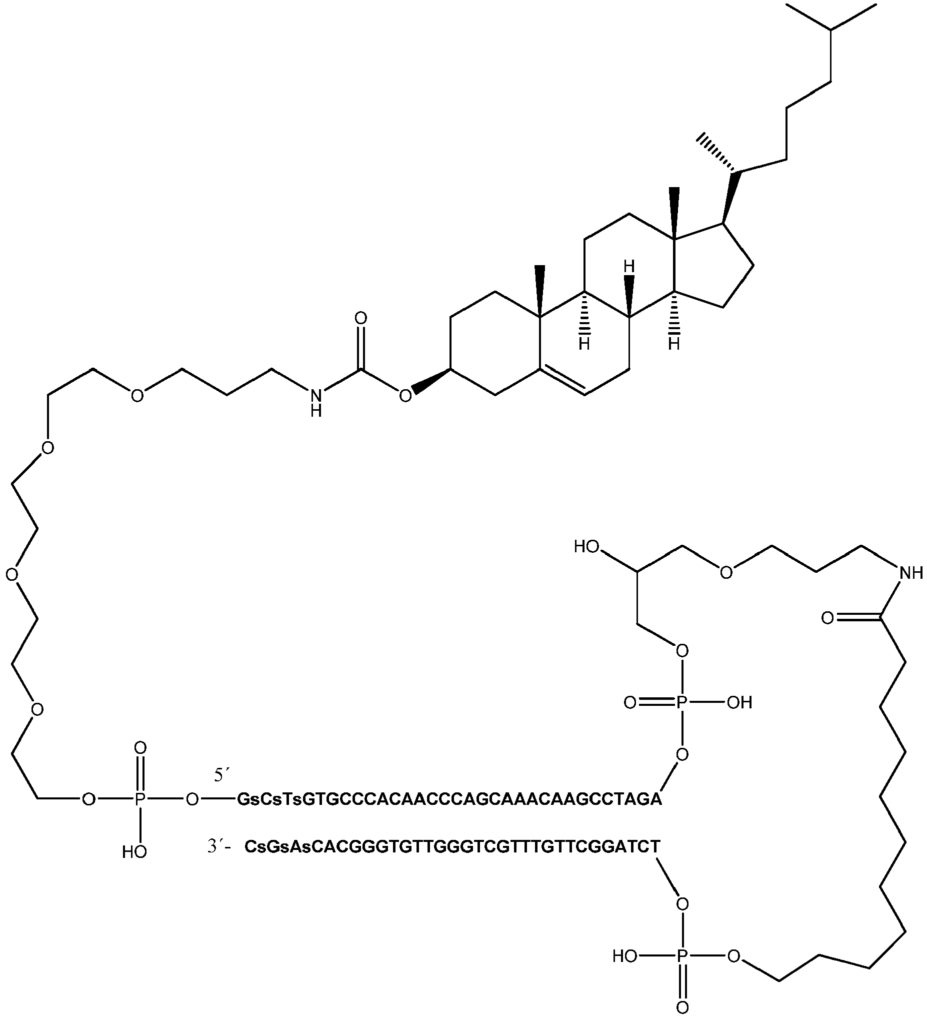

5. The pharmaceutical composition or the kit (kit-of-parts) according to anyone claims 1 to 4, wherein the HDAC inhibitor is selected from the group consisting of belinostat (PXD-101), vorinostat (SAHA), entinostat (MS-275) and romidepsin (FK-228).

6. The pharmaceutical composition or the kit (kit-of-parts) according to anyone claims 1 to 5, wherein the HDAC inhibitor is belinostat (PXD-101).

7. A pharmaceutical composition according to any one claims 1 to 6 for use in the treatment of a cancer.

8. A kit (kit-of-parts) comprising a Dbait molecule and a HDAC inhibitor and as a combined preparation for simultaneous, separate or sequential use, in particular for use in the treatment of cancer.

9. The pharmaceutical composition or the kit for use according to claims 7 or 8, wherein the cancer is selected from leukemia, lymphoma, sarcoma, melanoma, and cancers of the head and neck, kidney, ovary, pancreas, prostate, thyroid, lung, esophagus, breast, bladder, brain, colorectum, liver, and cervix.

10. The pharmaceutical composition or the kit for use according to claim 9, wherein the cancer is triple-negative breast cancer (TNBC).
